# EUROPÄISCHE PATENTANMELDUNG

(11) **EP 2 626 057 A1**
(43) Veröffentlichungstag der Anmeldung: **14.08.2013**
(21) Anmeldenummer: 12154653.5
(22) Anmeldetag: 09.02.2012
(51) Int. Cl.: A61K 6/00

(54) **Système adhésif enrichi en étain pour la liaison de matériaux de remplissage à l'aide des tissus durs de la dent et pour le revêtement des tissus durs de la dent**

(71) Anmelder: Justus-Liebig-Universität Gießen, 35390 Giessen (DE)
(72) Erfinder: Prof. Ganß, Carolina, 35037 Marburg (DE); Prof. Lussi, Adrian, 3076 Worb (CH)
(74) Vertreter: Stumpf, Peter

(57) **Zusammenfassung**

Die Erfindung betrifft ein Zinnangereichertes Adhäsivsystem zur Behandlung von überempfindlichen Zähnen und zum Verbund von Kompositen und Keramiken mit der Zahnhartsubstanz, insbesondere für eine verbesserte Haftung von Kompositen und Keramiken sowie anderen Dentalmaterialien auf durch Erosion vorbelasteten Zähnen und Beschichtungsmaterialien mit der speziellen Indikation Erosion/Zahnverschleiß.

## Beschreibung

Die Erfindung betrifft ein Zinnangereichertes Adhäsivsystem zur Behandlung von überempfindlichen Zähnen und zum Verbund von Kompositen und Keramiken mit der Zahnhartsubstanz, insbesondere für eine verbesserte Haftung von Kompositen und Keramiken sowie anderen Dentalmaterialien auf durch Erosion vorbelasteten Zähnen und Beschichtungsmaterialien mit der speziellen Indikation Erosion/Zahnverschleiß.

### Beschreibung und Einleitung des allgemeinen Gebietes der Erfindung

Die vorliegende Erfindung betrifft das Gebiet Gesundheitswesen/Mittel für Zahnheilkunde.

### Stand der Technik

Zahnfarbene Restaurationen mit Kompositen und Keramiken und Kunststoffe zur Behandlung von überempfindlichen Zähnen sind heute in breiter Anwendung. Besonders die Füllungstherapie mit Kompositen (Kunststoffen) hat die Versorgung mit Amalgam weitgehend verdrängt. Komposite müssen jedoch aufgrund ihrer Materialeigenschaften (Schrumpfung, Hydrophobie) mit speziellen Techniken an der Zahnhartsubstanz verankert werden. Dazu wird die Zahnoberfläche mit speziellen Primern und Adhäsiven vorbehandelt und mit dem Füllungsmaterial verklebt. Ziel ist, es eine möglichst gute Haftung und einen dauerhaften Verbund von Zahnhartsubstanz und Füllungsmaterial zu erzielen. Die Haftkraft hängt unter anderem davon ab, wie gut das Adhäsiv auf ultrastruktureller Ebene in die Zahnhartsubstanz penetrieren kann.

Bei Schmelz wird durch Säureeinwirkung, in der Regel durch Phosphorsäure, ein sog. retentives Ätzmuster erzeugt. Komposite können diese aufgeraute Oberfläche gut benetzen und sich bei der Aushärtung mikromechanisch verankern.

Bei Dentin wird ebenfalls mit sauren Präparaten vorbehandelt, anschließend wird jedoch noch eine weitere Vorbehandlung mit hydro- bzw. amphiphilen Kompositen erforderlich, bis die resultierende Oberfläche von dem hydrophoben Komposit-Füllungs- oder -Zementierungsmaterial benetzt werden kann.

Da auch moderne Komposite bei der Polymerisation um 2 bis 3 Volumenprozent schrumpfen, ist eine wirksame Verankerung an Schmelz und Dentin erforderlich, um Spaltenbildungen an Füllungsrand und Kavitätenboden zu verhindern. Dazu wurden in den letzten 20 Jahren Adhäsivsysteme entwickelt, die für eine verbesserte Retention der Füllung sowie für eine Stabilisierung von Restauration und Restzahnsubstanz sorgen.

Ein einmal erzielter Verbund kann jedoch durch die Aktivität körpereigener Enzyme desintegriert werden. Trotz der in den letzten Jahren stark weiter entwickelten Adhäsivsysteme treten bei bestimmten Patientengruppen, insbesondere bei Patienten mit Erosionen, hohe Misserfolgsraten von zahnfarbenen Restaurationen auf [D Bartlett, G Sundaram: Int J Prosthodont, 2006, 19(6), 613-7.]. Diese können sowohl auf einen primär schlechteren Verbund auf erodierten Zahnhartsubstanzen als auch auf eine raschere enzymatische Desintegration der initial erzielten Haftung zurückgeführt werden. Auf mit Erosion vorbelasteten Zähne sind Kunststoffbeschichtungen mit Materialien, die auf Adhäsivsystemen beruhen, bislang wenig dauerhaft und gehen relativ rasch wieder verloren [G Sundaram, R Wilson, TF Watson, D Bartlett: Operative Dentistry, 2007, 32, 539-543].

Bisher gibt es keine Adhäsive und Beschichtungsmaterialien mit der speziellen Indikation Erosion/Zahnverschleiß. Erosive Defekte wurden bisher mit den allgemein in der Zahnheilkunde verwendeten Adhäsivsystemen versorgt.

Die Erfinder der vorliegenden Erfindung haben gezeigt, dass die Haftkraft eines Adhäsivs auf erodierter Zahnhartsubstanz durch die Vorbehandlung mit einer Lösung aus Aminfluorid/Natriumfluorid/Zinnchlorid deutlich erhöht werden kann. Weiterhin haben die Erfinder gezeigt, dass der Einsatz einer sauren Zinnchlorid-Lösung als Ätzmittel in klinisch üblicher Anwendungszeit zu den für den adhäsiven Verbund erforderlichen strukturellen Veränderungen in Schmelz (retentives Ätzmuster) und Dentin (Entfernung des Smear layers) und zu einer Anreicherung von Zinn in Schmelz und Dentin führt. Die Erfinder haben außerdem gezeigt, dass die Beimischung von Zinnchlorid zu Primern zu einer Anreicherung von Zinn in Schmelz und Dentin führt..

### Aufgabe

Aufgabe der vorliegenden Erfindung ist es, Adhäsivsysteme und Beschichtungsmaterialien bereitzustellen, mit denen ein besserer und dauerhafterer Verbund von (erodierter) Zahnhartsubstanz und zahnfarbenen Füllungsmaterialien erreicht wird.

### Lösung der Aufgabe

Gelöst wird diese Aufgabe durch ein Stoffgemisch umfassend eine Komponente 1 und Komponente 2, wobei Komponente 1 ein Zinnsalz ist und Komponente 2 ausgewählt ist aus
a. wässrige Lösung, die zur Vorbehandlung vor Zahnrestaurationen eingesetzt wird
b. Ätzmittel
c. Primer
**d.** Bond
**e.** Kunststoff-Komposit
f. einem Adhäsivsystem umfassend mindestens ein Element, ausgewählt aus Ätzmittel, Primer und Bond

Den Kern der Erfindung bildet das überraschende Ergebnis, dass die Zugabe von Zinn-Ionen zu einer beliebigen Komponente 2a bis 2f zu einer verbesserten Haftung des Komposits auf erodierter Zahnhartsubstanz führt. In einer vorteilhaften Ausführungsform sind die Komponenten 2a bis 2f kommerziell erhältlich.

Das erfindungsgemäße Verfahren besteht darin, dass ein Zinnsalz (Komponente 1) mit einer beliebigen Komponente 2a bis 2f zu mischen. In einer vorteilhaften Ausführungsform erfolgt die Zinnbeimengung zur Komponente 2 vor der Auftragung auf den Zahn. Alternativ dazu wird die Zinnbeimengung zur Komponente 2 während der Auftragung auf den Zahn durchgeführt.

Das erfindungsgemäße Gemisch, das Verfahren zu seiner Herstellung sowie die Verwendungen dieses Gemisches sind nachfolgend erläutert. Die Erfindung ist nicht auf eine der nachfolgend beschriebenen Ausführungsformen beschränkt, sondern in vielfältiger Weise abwandelbar. Sämtliche aus den Ansprüchen und der Beschreibung hervorgehenden Merkmale und Vorteile, einschließlich konstruktiver Einzelheiten, Verfahrensschritte und der Reihenfolge von Verfahrensschritten, können sowohl für sich als auch in den verschiedensten Kombinationen erfindungswesentlich sein.

Ein Adhäsivsystem ist eine dem Fachmann bekannte Haftvermittlung zwischen Komposit und Zahnhartsubstanz. Ein Komposit ist ein Füllungs- oder Beschichtungsmaterial, das vom Fachmann standardgemäß für die Restauration von Zahnhartsubstanzdefekten eingesetzt wird. Die Zinnbeimengung kann zu einer oder mehreren der verschiedenen Elemente von kommerziell erhältlichen Adhäsivsystemen oder Kunststoffbeschichtungen erfolgen, die weiter unten aufgelistet sind. Ein Adhäsivsystem umfasst mindestens eines der Elemente Ätzmittel, Primer, Adhäsiv, Bond. Ein Ätzmittel ist eine saure Vorbehandlungskomponente, die im Schmelz ein retentives Ätzmuster erzeugt welches der mikromechanischen Verankerung des Adhäsivs dient, und im Dentin den Smear layer entfernt, was die Penetration von Primern und Adhäsiven ermöglicht. Das Ätzmittel kann als Flüssigkeit oder in Form eines Gels zum Einsatz kommen.

In einer vorteilhaften Ausführungsform handelt es sich bei der in Anspruch 1 genannten Komponente 2 um ein Ätzmittel in Form einer wässrigen Lösung mit einem pH zwischen 1 und 5. Besonders vorteilhaft handelt es sich um eine wässrige Phosphorsäurelösung mit einem pH zwischen 1 und 5. Das Ätzmittel kann sowohl als Bestandteil eines Adhäsivsystems vorliegen als auch für sich alleine. Bei der Restauration von Dentin wird das Ätzmittel meist als Bestandteil eines Adhäsivsystems eingesetzt, während es bei der Restauration von Schmelz in den meisten Fällen für sich alleine ohne Adhäsivsystem eingesetzt wird. Bei der Restauration von Schmelz erfolgt in der Regel direkt nach der Konditionierung mit dem Ätzmittel das Auftragen des Komposits. Die Zinnzugabe kann zu kommerziell erhältlichen Primern erfolgen.

In einer anderen vorteilhaften Ausführungsform handelt sich bei der in Anspruch 1 genannten Komponente 2 um einen Primer. Ein Primer ist eine hydrophile Komponente, die den Übergang vom hydrophilen Dentin zum hydrophoben Komposit herstellt und die oberste Schicht des hydrophilen Dentins oder Schmelz penetriert. Die Zinnzugabe kann zu kommerziell erhältlichen Primern erfolgen.

In einer weiteren vorteilhaften Ausführungsvorm handelt es sich bei der in Anspruch 1 genannten Komponente 2 um ein Adhäsiv. Ein Adhäsiv ist eine amphiphile Komponente, die den Übergang vom hydrophilen Primer zum hydrophoben Komposit herstellt.

In einer weiteren vorteilhaften Ausführungsvorm handelt es sich bei der in Anspruch 1 genannten Komponente 2 um ein Bond. Ein Bond ist ein Haftvermittler zwischen Adhäsiv und Komposit.

In einer weiteren vorteilhaften Ausführungsvorm handelt es sich bei der in Anspruch 1 genannten Komponente 2a um Wasser. In einer besonders vorteilhaften Ausführungsform handelt es sich bei Komponente 2a um ein kommerziell erhältliches Mundwasser.

Die Zugabe von Zinn-Ionen erfolgt durch Vermischen des Zinnsalzes mit mindestens einer der Komponenten 2 a) bis f). In vorteilhafter Ausführungsform erfolgt die Zinnbeimengung in Form einer wässrigen Zinnsalz-Lösung zur Komponente 2. In besonders vorteilhafter Ausführungsform erfolgt die Zinnbeimengung vor der Auftragung auf den Zahn.

Das Zinnsalz ist ausgewählt aus Zinnhalogeniden, Zinnsulfat, Zinn-II-phosphat und Zinnoxalat. Der Gesamtanteil der wässrigen Zinnsalz-Lösung soll 0,5 bis 80 Gewichtsprozent des erfindungsgemäßen Gesamtgemisches ausmachen, bevorzugt 1 bis 30 Gewichtsprozent, besonders bevorzugt 4 bis 20 Gewichtsprozent. Die Auftragung der erfindungsgemäßen Zinn-gedopten Adhäsivsystem-Komponente auf den Zahn erfolgt wie klinisch üblich.

Es gibt verschiedene Adhäsivsysteme, wie z.B. die Mehrschritt-Adhäsivsysteme oder die Einflaschensysteme. Im Folgenden ist der prinzipielle Aufbau von verschiedenen Adhäsivsystemen, die vom Fachmann auch als Bondingsysteme benannt werden, aufgelistet. Alle Bondingsysteme eignen sich für die erfindungsgemäße Zinnbeimengung. Dabei kann die Zinnzugabe zu jeder der aufgelisteten Komponenten eines Adhäsivsystems erfolgen, bevorzugt aber zu den hydrophilen Komponenten, besonders bevorzugt zum Ätzmittel oder Primer. Innerhalb einer Zahnrestauration kann die Zinnbeimengung zu einer oder mehreren Komponenten des Adhäsivsystems erfolgen.

Die nachfolgend aufgeführten Techniken gehören alle zur Gruppe der Adhäsivsysteme. Adhäsivsysteme werden vom Fachmann alternativ auch als Bondingsystem benannt.

### 3(4)-Schritt-selektive Ätztechnik umfasst folgende 4 Komponenten:

1. **Ätzmittel:** zur selektiven Ätzung des Zahnschmelz, vorzugsweise mit Phosphorsäure
2. **Primer:** hydrophiles, saures Monomer diffundiert in Dentin und ätzt dieses
3. **Ädhäsiv:** amphiphil - infiltriert die Oberfläche des Dentins
4. **Bond:** Haftvermittlung zum Komposite

**Primer,** die zur Dentin-Konditionierung eingesetzt werden, bestehen üblicherweise aus Wasser, Glutaraldehyd, Maleinsäure und PEMA (Polyethylmethacrylat). Das in Schritt 3 eingesetzte Adhäsiv dient der Benetzung des Dentins. Es besteht bevorzugt aus Wasser, Azeton, Maleinsäure und Dimethacrylat. Das in Schritt 4 eingesetzte Bond, besteht bevorzugt aus Dimethacrylat, Bis-GMA Initiatoren und Stabilisatoren, besonders bevorzugt aus MDP (10-Methacryloyloxydecyl-dihydrogenphosphat), HEMA (2-Hydroxyethyl-)methacrylat), Bis-GMA (Bis-Phenol A Diglycidylmethacrylat), hydrophobes Dimethacrylat, Di-Camphorquinon.

Die erfindungsgemäße Zinn-Beimischung kann bei der 3(4)-Schritt-selektiven Ätztechnik zum Ätzmittel, Primer oder Adhäsiv erfolgen, bevorzugt zum Ätzmittel oder Primer, ganz besonders bevorzugt zur Phosphorsäure oder zum hydrophilen sauren Monomer.

### 3-Schritt - Totalätztechnik:

1. Ätzmittel: für simultane Konditionierung (Ätzung) von Schmelz und Dentin
2. Hydrophiler Primer - Infiltration in demineralisiertes Dentin
3. Hydrophobes oder amphiphiles Adhäsiv

Im ersten Schritt kommt es durch Einsatz des Ätzmittels, meist einer Phosphorsäure in Form eines Gels zur Entfernung der Schmierschicht. Durch 15 bis 60 Sekunden langes Einwirken der Säure wird ein Ätzmuster erzielt.

Im zweiten Schritt erfolgt die Applikation des hydrophilen Primers. Dieses ist meist ein hydrophiles Monomer, welches in das konditionierte Kollagennetzwerk penetriert, wobei eine mikromechanische Verankerung in der sogenannten Hybridschicht erreicht wird. Die Zinn-Verbindung kann bei 3-Schritt-Totalätztechniken dem Ätzmittel oder/und dem hydrophilen Primer beigefügt werden. Die Auftragung der Zinn-gedopten Ätzmittel bzw. Primer erfolgt standardgemäß wie oben beschrieben, bzw. wie vom Hersteller empfohlen. Das Adhäsiv ist der eigentliche Haftvermittler, der die Verbindung zwischen dem Komposit und dem mit Konditionierer und/oder Primer vorbehandelten Dentin herstellt. Die Penetration des Adhäsivs in das mit Primer behandelte demineralisierte Dentin führt zur Bildung der Hybridschicht und das Einfließen in die eröffneten Tubuli zur Entstehung der Kunststoff-Tags. Auf diese Weise wird eine wirkungsvolle Versiegelung des Dentins erzielt. Um möglichst vollständig in das demineralisierte Dentin eindringen zu können, benötigt das Adhäsiv ausreichend Zeit (etwa zehn Sekunden) und sollte dann nicht durch Luftbläser extrem ausgedünnt werden. Um den Polymerisationsschrumpfungskräften besser entgegenwirken zu können, sollten die Adhäsivsysteme ihre volle Wirkung im Dentin aufgebaut haben. Dazu sollten sie vor der Applikation des Füllungsmaterials separat lichtgehärtet werden.

Die erfindungsgemäße Zinn-Beimischung kann bei der 3-Schritt-selektiven Ätztechnik zum Ätzmittel, Primer oder Adhäsiv erfolgen, bevorzugt zum Ätzmittel oder Primer, ganz besonders bevorzugt zum Ätzmittel.

### 2-Schritt-Totalätztechnik

1. Ätzmittel für simultane Konditionierung von Schmelz und Dentin
2. Primer und Adhäsiv in Einem (wird auch "Primer-Adhäsiv" genannt)

Im Falle von **Primer-Adhäsiven** (Zwei-Schritt-Systemen) muss die Druckluft vorsichtig dosiert werden, damit die aufgetragene Lösung nicht zerstäubt und eine durchgehende Filmbildung sichergestellt wird. Die Vorbehandlung mit **Primern** dient dazu, die Oberflächenenergie des Dentins zu erhöhen und seine Benetzung mit dem anschließend applizierten Adhäsiv zu verbessern. Nach der Verdunstung des Lösungsmittels bleibt ein dünner Monomerfilm zurück, der an der Oberfläche haftet. Daher müssen die Monomermischung und das Lösungsmittel so aufeinander abgestimmt sein, dass eine Infiltration in das Kollagengeflecht optimal erfolgen kann. Diese Infiltration in das Kollagennetzwerk kann durch ein übermäßiges Trocknen des Dentins nach der Konditionierung eingeschränkt werden, da das Kollagennetzwerk beim Austrocknen zusammenfällt.

Die erfindungsgemäße Zinn-Beimischung kann bei der 2-Schritt-Totalätztechnik zum Ätzmittel oder Primer-Adhäsiv erfolgen, bevorzugt zum Ätzmittel.

### Selbstkonditionierende Adhäsivsysteme

Bei diesen so genannten All-in-one-Adhäsiven wird nur eine Lösung appliziert, zu welcher die Zinnbeimengung vor der Auftragung auf den Zahn erfolgen sollte. All-in-one-Adhäsive enthalten eine ausgewogene Mischung von hydrophilen und hydrophoben Monomeren sowie Monomere mit Säurerestern und erfüllen so neben der Funktion des Adhäsivs gleichzeitig auch die des Ätzmittels und des Primers. Während die ersten All-in-one-Adhäsive aus zwei Fläschchen gemischt und in zwei Lagen aufgetragen und ausgehärtet werden mussten, genügt bei neueren Präparaten eine einmalige Applikation. Einige davon werden nach wie vor aus zwei Lösungen angemischt, von denen eine die Monomere und die andere hauptsächlich Wasser enthält. Diese Darreichungsform ist erforderlich, wenn die selbstkonditionierenden Monomere im sauren Milieu nicht ausreichend hydrolysestabil sind. Erst bei der Mischung mit Wasser dissoziieren sie unter Freisetzung von H+-Ionen. Noch weiter vereinfacht ist die Anwendung von gebrauchsfertigen Lösungen für die Einmalapplikation, in denen das für die Dissoziation der Säuregruppen erforderliche Wasser bereits enthalten ist. Für diese Art der Darreichung ist es wiederum erforderlich, dass die selbstkonditionierenden Monomere ausreichend hydrolysestabil sind. Um eine optimale Ätzwirkung sicherzustellen, wird empfohlen, selbstkonditionierende Primer und All-in-one-Adhäsive lange genug einwirken zu lassen (etwa 30 Sekunden) und durch leichtes "Einreiben" in Bewegung zu halten. Dadurch wird verhindert, dass die sauren Lösungen zu schnell neutralisiert werden und in ihrer Wirksamkeit nachlassen.

Die erfindungsgemäße Zinn-Beimischung erfolgt bei allen oben beschriebenen kommerziell erhältlichen Adhäsivsystemen jeweils zu einer oder mehreren Komponenten, ausgewählt aus Ätzmittel, Primer, selbstätzender bzw. selbstkonditionierender Primer, Primer-Adhäsiv, Adhäsiv und Bond innerhalb einer Zahnrestauration.

Die erfindungsgemäßen Gemische sind für Zahnrestaurationen verwendbar. In einer vorteilhaften Ausführungsform werden die erfindungsgemäßen Stoffgemische bei Zahnrestaurationen von erodierten Zähnen oder Zähnen mit nicht kariesbedingten Zahnartsubstanzerkrankungen verwendet. In einer weiteren vorteilhaften Ausführungsform werden die erfindungsgemäßen Stoffgemische bei der Zahnbehandlung zur Vorbeugung vor weiteren Erosionen verwendet.

Im Folgenden wird die Erfindung anhand von Beispielen näher beschrieben, wobei diese als Ausführungsbeispiele und in keiner Weise limitierend zu verstehen sind.

### 1. Präparation eines extrakorporalen Dentin-Probensatzes

Extrahierte menschliche Backenzähne werden unter fließendem Wasser gereinigt und in 0,5 % Chloramin-Lösung bei 4 °C bis zur Verwendung gelagert. Sie werden in kreisförmige Formen mit selbsthärtenden Acrylharz (Paladur, Heraeus Kulzer GmbH, Hanau) eingebettet und mit einer Schleifmaschine und Siliziumcarbid (SiC) Papier (Struers L aboPoi-21/SiC # 320, Struers, Ballerup, Dänemark) solange geschliffen, bis die gesamte Oberfläche aus koronalem Dentin besteht. Die Dentin-Oberfläche wird luftgetrocknet und sorgfältig auf das Fehlen von Zahnschmelz überprüft. Zähne mit freigelegter Pulpa werden verworfen. Zum Schluss werden alle Zähne für 15 Sekunden mit SiC Papier (Struers LaboPoi-21/SiC #500, Struers) geschliffen. Diese Proben werden den Versuchsbedingungen ausgesetzt, anschließend wird das zu untersuchende Adhäsiv-/Kompositsystem angewendet. Für die anschließende Haftungsmessung werden aus diesen Proben mittels einer Diamantsäge und digitalem Messschieber jeweils mehrere Proben einheitlicher Größe (1,05mm x 1,05 mm) hergestellt, die in die Vorrichtung einer Abzugsvorrichtung eingebracht werden können.

### 2. Präparation eines extrakorporalen Schmelz-Probensatzes

Extrahierte menschliche Molaren werden unter fließendem Wasser gereinigt und in 0,5 % Chloramin-Lösung bei 4 °C bis zur Verwendung gelagert.

Sie werden in kreisförmige Formen mit selbsthärtenden Acrylharz (Paladur, Heraeus Kulzer GmbH, Hanau) eingebettet und mit einer Schleifmaschine und Siliziumcarbid (SiC) Papier (Struers LaboPoi-21/SiC # 320, Struers, Ballerup, Dänemark) solange geschliffen, bis eine plane Versuchsfläche von etwa 4 mm x 4 mm entsteht. Zum Schluss werden alle Zähne für 15 Sekunden mit SiC Papier (Struers LaboPoi-21 /SiC #500, Struers) geschliffen. Diese Proben werden den Versuchsbedingungen ausgesetzt, anschließend wird das zu untersuchende Adhäsiv-/Kompositsystem angewendet. Für die anschließende Haftungsmessung werden aus diesen Proben mittels einer Diamantsäge und digitalem Messschieber jeweils mehrere Proben einheitlicher Größe (1,05mm x 1,05 mm) hergestellt, die in die Vorrichtung einer Abzugsvorrichtung eingebracht werden können.

### 3. Erosive Demineralisierung

Die klinische Situation von erosiven Defekten wird simuliert durch eine 10-tägige zyklische De- und Remineralisierungsprozedur. Dazu werden die Proben 6 mal pro Tag jeweils 5 Minuten in 0,05 M Zitronensäure pH 2.3 im Schüttelbad inkubiert. In den Zwischenzeiten wird jeweils 60 Minuten remineralisiert in 4.08mM H3P04, 20.10mM KCI, 11.90mM Na2C03, 1.98mM CaCl2, pH 6.7.

### 4. Aufbringen des Komposits über ein Adhäsivsystem

**Primer:** 10-methacryloyloxydecyl Dihydrogen Phosphate (MDP), 2-Hydroxyethyl Methacrylat (HEMA), hydrophiles Dimethacrylat, Dicamphorquinone, Wasser) **Bond:** MDP, HEMA, Bisphenolglycidylmethacrylat (Bis-GMA), hydrophobes Dimethacrylat, Di-Camphorquinon.

Nach der Adhäsiv-Behandlung werden 2 Schichten mit je 2 mm Komposit (bestehend aus Bis-GMA, Urethan-Dimethacrylat (UDMA), Bisphenol-A-Polyethylen-Glycoldiether-Dimethacrylat (Bis-EMA)) aufgetragen, und jeweils 20 Sekunden mit LED-Licht ausgehärtet. Die mit Komposit beschichteten Zähne werden 24h bei 100% Luftfeuchtigkeit und 37°C gelagert.

### 5. Haftungsmessung des Füllmaterials an der Zahnhartsubstanz

Die wie in Beispiel1 beschrieben hergestellten Proben einheitlicher Größe werden in einer Spannvorrichtung fixiert und eine Zugkraft von 1,00 mm/min angelegt. Die maximale Zugkraft (Fₘₐₓ (N)) wird gemessen und daraus die Haftungskraft in µTBS (Micotensile bond strength) errechnet: µTBS= F ₘₐₓ/BSU. (N= Newton, BSU = bonded surface (mm²)).

### Ausführungsbeispiel 6

### (Vorbehandlung mit einer Sn/F Mundspüllösung)

Behandlung der extrakorporalen Zähne in aufeinander folgenden Schritten: Herstellung von Proben wie in Beispiel 1 beschrieben. Zyklische De- und Remineralisierung (entsprechend Beispiel 2). 20 Proben der Gruppe 1: Behandlung mit Sn/F-Mundspüllösung (125 ppm F als Aminfluorid 375 ppm F als NaF, und 800 ppm Sn als SnC1₂, pH 4.5), jeweils 2 Minuten vor und nach dem letzten Demineralisierungsschritt. Für 20 Proben der Gruppe 2 (Vergleichsproben) fällt dieser Behandlungsschritt weg. Bei allen 40 Proben: Aufbringung des Adhäsivsystems (entsprechend Beispiel 4). Haftungsmessung (entsprechend Beispiel 5). Ergebnis: Die mit Sn/F-Mundspüllösung vorbehandelten Proben zeigen deutlich bessere Haftung des Komposits im Vergleich zu den Proben ohne Sn/F- Behandlung.

### Ausführungsbeispiel 7

### (saure Zinnchlorid-Lösung als Ätzmittel für Schmelz und Dentin)

Gruppe 1: Schmelz- und Dentinproben werden nach Beispiel 1 und 2 präpariert und nach Beispiel 3 erosiv demineralisiert. Danach werden sie mit einer Phosphorsäure/SnF₂ Lösung (35%ige Phosphorsäure + 250 ppm Zinnfluorid) für 30 Sekunden behandelt und anschließend für 60 Sekunden mit Leitungswasser abgespült. Gruppe 2 (Vergleichsproben): Schmelz- und Dentinproben werden nach Beispiel 1 und 2 präpariert und mit 35 %iger Phosphorsäure für 30 Sekunden behandelt und anschließend für 60 Sekunden mit Leitungswasser abgespült. Bei allen Proben aus Gruppe 1 und 2 in aufeinander folgenden Schritten: Aufbringung des Adhäsivsystems und des Komposits (entsprechend Beispiel 4). Haftungsmessung (entsprechend Beispiel 5). Ergebnis: Die Komposithaftung der Proben aus Gruppe 1 ist an Schmelz vergleichbar mit Gruppe 2 (kein Verlust an Haftung gegenüber Standard) und bei Dentin deutlich höher als die Komposithaftung der Proben aus Gruppe 2.

### Ausführungsbeispiel 8

### (Zinn-Zugabe zum Primer eines Mehrschrittadhäsivsystems)

Gruppe 1: Dentinproben werden nach Beispiel 1 präpariert und mit einem Mehrschrittadhäsivsystem behandelt. Im ersten Schritt werden die Proben für 30 Sekunden mit Phosphorsäure (35%) behandelt und anschließend für 60 Sekunden mit Leitungswasser abgespült. Anschließend wird der mit Zinn angereicherte Primer (z.B. HEMA, GPDM, PAMA, KC, Ethanol, Wasser mit 35%, 17,5%, oder 10% SnCl₂ oder mit 35%, 17,5% oder 10% SnF₂ aufgetragen und nach 15 Sekunden verblasen. Dann wird das Adhäsiv aufgetragen (z.B. Bis-GMA, HEMA, GDMA, anorganische Füller), dünn verblasen und lichtgehärtet. Gruppe 2 (Vergleichsproben): die Proben werden mit dem entsprechenden Primer und Adhäsiv ohne Zinn behandelt.

Nach Beispiel 4 wird Komposit aufgebracht (Gruppe 1 und 2) und die Haftkraft nach Beispiel 5 bestimmt. Ergebnis: Die Komposithaftung bei Proben der Gruppe 1 ist höher als bei Proben der Gruppe 2.

HEMA: Hydroxyethyl-Methacrylat, GPDM: Glycerolphosphatdimethacrylat, PAMA: Phthalsäuremomethacrylat, GDMA: Glycerol-dimethacrylat

### Ausführungsbeispiel 9

### (Zinn-Zugabe zum Primer eines Einflaschenadhäsivsystems)

Gruppe 1: Dentinproben werden nach Beispiel 1 präpariert und mit einem zinnangereicherten Einflaschenadhäsiv behandelt (z.B. Methacrylat-Phosphorester, Bis-GMA, Initiatoren, Stabilisatoren, 2-Hydroxyethyl-Methacrylat (HEMA)) angereichert mit 35%, 17,5%, 10% SnCl₂ oder niedriger konzentriert oder mit 35%, 17,5%, 10% SnF₂ oder niedriger konzentriert aufgetragen und lichtgehärtet. Gruppe 2 (Vergleichsproben): die Proben werden mit dem entsprechenden Einflaschenadhäsiv ohne Zinn behandelt.

Nach Beispiel 4 wird Komposit aufgebracht (Gruppe 1 und 2) und die Haftkraft nach Beispiel 5 bestimmt. Ergebnis: Die Komposithaftung bei Proben der Gruppe 1 ist höher als bei Proben der Gruppe 2.

### Ausführungsbeispiel 10

### Anwendung auf erodiertem Dentin

Dentinproben werden nach Beispiel 3 und 4 erosiv demineralisiert um die klinische Situation bei Patienten mit Erosionen zu simulieren. Diese Proben werden entsprechend den Ausführungsbeispielen 7, 8, 9 und 10 behandelt und die Haftung des Komposits entsprechend Beispiel 5 bestimmt. Ergebnis: Die Haftung von Komposit an erodierten Proben ist nach Anwendung von Adhäsivsystemen mit Zinnanreicherung höher als nach Anwendung entsprechender zinnfreier Adhäsivsysteme.

## Patentansprüche

1. Stoffgemisch umfassend eine Komponente 1 und Komponente 2, wobei Komponente 1 ein Zinnsalz ist und Komponente 2 ausgewählt ist aus
a. wässrige Lösung, die zur Vorbehandlung vor Zahnrestaurationen eingesetzt wird
b. Ätzmittel
c. Primer
d. Bond
e. Kunststoff-Komposit
f. einem Adhäsivsystem umfassend mindestens ein Element, ausgewählt aus Ätzmittel, Primer und Bond

2. Stoffgemisch gemäß Anspruch 1, **dadurch gekennzeichnet, dass** das Zinnsalz als wässrige Zinnsalz-Lösung vorliegt und 0,5 bis 80 Gewichtsprozent des Gesamtgemisches ausmacht.

3. Stoffgemisch gemäß einem der Ansprüche 1 bis 2, **dadurch gekennzeichnet, dass** das Zinnsalz ausgewählt ist aus Zinnhalogeniden, Zinnsulfaten, Zinn-II-phosphat und Zinnoxalat.

4. Stoffgemisch gemäß einem der Ansprüche 1 bis 3, **dadurch gekennzeichnet, dass** das Adhäsivsystem ausgewählt ist aus der 3-Schritt-Totalätztechnik, 3(4)-Schritt-Selektive Ätztechnik, 2-Schritt-Totalätztechnik oder All-In-One-Techniken

5. Stoffgemisch gemäß einem der Ansprüche 1 bis 4 **dadurch gekennzeichnet, dass** Komponente 2 ein Ätzmittel ist, wobei es sich um wässrige Lösungen mit einem pH zwischen 1 und 5 handelt.

6. Verfahren zur Herstellung des Stoffgemisches gemäß einem der Ansprüche 1 bis 5, **dadurch gekennzeichnet, dass** die Komponente 1 mit der Komponente 2 vermischt wird.

7. Verwendung von Stoffgemischen gemäß einem der Ansprüche 1 bis 6 bei Zahnrestaurationen.

8. Verwendung von Stoffgemischen gemäß einem der Ansprüche 1 bis 7, **dadurch gekennzeichnet, dass** die Zahnrestauration auf erodierten Zähnen oder Zähnen mit anderen nicht-kariesbedingten Zahnhartsubstanzerkrankungen erfolgt.

9. Verwendung von Stoffgemischen gemäß einem der Ansprüche 1 bis 8, **dadurch gekennzeichnet, dass** die Zahnrestauration zur Vorbeugung vor weiterer Erosion erfolgt.
